# EUROPEAN PATENT SPECIFICATION

(11) **EP 2 065 708 B1**
(45) Date of publication and mention of the grant of the patent: **01.01.2014**
(21) Application number: 08020603.0
(22) Date of filing: 27.11.2008
(51) Int. Cl.: G01N 33/92

(54) **Method for measuring high-density lipoprotein cholesterol**
Verfahren zur Messung von hochdichtem Lipoproteincholesterol
Procédé de mesure de cholestérol de lipoprotéine à forte densité

(30) Priority: 28.11.2007 JP 2007306715; 28.11.2007 JP 2007306716
(43) Date of publication of application: 03.06.2009
(73) Proprietor: FUJIFILM Corporation, Tokyo 106-8620 (JP)
(72) Inventor: Inomata, Hiroko, Asaka-shi Saitama 351-8585 (JP); Kageyama, Shigeki, Asaka-shi Saitama 351-8585 (JP)
(74) Representative: Albrecht, Thomas

(56) References cited:
- EP-A- 1 854 894
- EP-A- 1 982 726
- GB-A- 1 022 878
- JP-A- 5 341 456
- JP-A- 52 046 816

## Description

### Technical Field

The present invention relates to a method for measuring high-density lipoprotein cholesterol (HDL-C) in a specimen and a polyglycidol compound useful for said method or as a surfactant.

### Background Art

Lipids existing in blood are incorporated into the structures of lipoproteins except that free fatty acids are bound to albumin, and they exist as chylomicron (CHM), very low-density lipoprotein (VLDL), low-density lipoprotein (LDL), high-density lipoprotein (HDL) . Cholesterol is distributed especially in VLDL, LDL and HDL. Since HDL is considered as a prophylaxis factor of heart diseases due to arteriosclerosis, it is known that measurement of blood level of high-density lipoprotein cholesterol (HDL-C), instead of HDL, provides a useful criterion for predicting onset of arteriosclerotic diseases.

At present, ultracentrifugation method, electrophoresis method, and precipitation method are widely known as measurement methods of HDL cholesterol. However, the ultracentrifugation method requires long time for separation, and ultracentrifugation apparatuses themselves are expensive so that low cost measurement cannot be expected, and therefore the method is unsuitable for routine tests. The electrophoresis method still has problems concerning quantification, for example, difference of electrophoresis supporting.medium causes difference of separation performance, and depending on condition for use thereof or type of detection reagent used, difference in measurement results may be caused.

The precipitation method is a method of precipitating CHM, LDL and VLDL using a combination of a polyanion and a divalent metal ion as a precipitation reagent, and measuring cholesterol in HDL, i.e., HDL-cholesterol, remaining in the supernatant using a chemical reagent or an enzyme. As the precipitation reagent, combination systems of a polysaccharide sulfate and an alkaline earth metal ion or a divalent metal ion other than alkaline earth metal, which was well known from early 1960s, inorganic polyanion salt systems, polyethylene glycol, and the like are widely used (Nakai T., HDL-Metabolism, Assay Methods and Clinical Application), Chugai Igaku-Sha, 1986). Specific examples of the precipitation reagent include, for example, heparin-calcium type reagents, dextran sulfate-magnesium type reagents, phosphotungstic acid-magnesium type reagents.

In these precipitation methods, serum and a precipitation reagent are mixed and left for a given period of time, and subjected to centrifugation at 3000 rpm, then a given volume of the supernatant is separated and used to perform a chemical reaction or an enzymatic reaction and thereby quantify HDL-cholesterol. However, this procedure has problems resulting from the precipitation reagent, problems concerning centrifugation operation. For example, as improvements of the precipitating agent for increasing precipitation efficiency, methods disclosed in Japanese Patent Unexamined Publication (KOKAI) Nos. 55-78254, 55-93065, 61-263467, 62-19768, Japanese Patent Publication (KOKOKU) No. 1-39553, have been proposed.

It is known that, in the conventional precipitation methods, when a reagent containing much triglycerides is used, a part of precipitates float after the centrifugation, and thus they also have a problem that adjustment of centrifugation conditions is additionally needed in order to avoid the problem. Moreover, the method of using phosphotungstic acid salt-magnesium ion has also a problem that production of precipitates may vary depending on pH of the solution, and thus strict pH adjustment become necessary. Furthermore, when the supernatant of the centrifuged solution is separated, and especially when volume of the supernatant is small, it is difficult to determine the border of the precipitates by visual inspection, and therefore there arise problems concerning reproducibility or precision, or individual differences may occur to degrade precision of the quantitative analysis. Therefore, it is desired to provide a means for improving these drawbacks concerning centrifugation operation.

In recent years, direct methods not requiring these complicated operations and usable in automatic analyzers have been rapidly spreading. For example, Japanese Patent Unexamined Publication No. 8-131197 discloses a method for measuring cholesterol in HDL in which sulfated cyclodextrin used as a coagulant is sufficiently reacted with lipoproteins other than HDL, and then an enzyme modified with polyoxyethylene glycol is made to react thereon. International Publication WO98/26090 discloses a method in which lipoproteins other than HDL are eliminated with a catalase in the first step, and HDL-C is measured by using an activator which specifically reacts on HDL in the second step. Japanese Patent Unexamined Publication No. 9-96637 describes a method in which antibodies directed to lipoproteins other than HDL is first made to react, then HDL is dissolved, and cholesterol in HDL is measured. Japanese Patent Unexamined Publication No. 11-56395 describes a method of measuring cholesterol in HDL by using a combination of an HDL-soluble surfactant and a dissolution inhibitor for lipoproteins other than HDL. International Patent Publication WO04/48605 describes a method of measuring lipids in a specific lipoprotein by using a polycyclic polyoxyalkylene derivative. However, in these methods, in order to suppress reactions with lipoproteins other than HDL, expensive reagents such as enzymes modified with PEG and antibodies must be used.

The precipitation method is also mainly used in the field of dry chemistry, and a novel dry type test piece utilizing the direct method has been developed in recent years (Japanese Patent No. 3686326). Japanese Patent Unexamined Publication No. 2005-137360 describes that selectivity is improved by using lipase derived from *Candida rugosa*. However, cholesterol in lipoproteins other than HDL cannot be completely removed in any of such test pieces.

Further, a water soluble compound comprising phenyl group substituted with 8 to 18 alkyl chains is known as a phenyl ether polyglycidol usable as a surfactant (GB1022878). However, this compound has a problem of low affinity for aromatic organic substances, since the hydrophobic portion of the compound is an alkyl chain, and it is highly concerned that it may act as an environmental hormone since it has an alkylphenol structure. Although a polyglycidol dodecyl ether is also known (Sagitani, H. et al., JAOCS, Vol. 66, pp.146-152, 1989), this compound has a problem that it requires much time for synthesis thereof because the compound is produced by binding glycidol one by one, and therefore the compound is not suitable for industrial applications. Therefore, it is desired to provide a surfactant which can be used instead of these compounds.
[Non-patent document 1] Nakai T., HDL-Metabolism, Assay Methods and Clinical Application), Chugai lgaku-Sha, 1986
[Non-patent document 2] Sagitani, H., et al., JAOCS, Vol. 66, pp.146-152, 1989 [Patent document 1] Japanese Patent Unexamined Publication 55-78254
[Patent document 2] Japanese Patent Unexamined Publication No. 55-93065
[Patent document 3] Japanese Patent Unexamined Publication No. 61-263467
[Patent document 4] Japanese Patent Unexamined Publication No. 62-19768
[Patent document 5] Japanese Patent Publication No. 1-39553
[Patent document 6] Japanese Patent Unexamined Publication No. 8-131197
[Patent document 7] International Patent Publication WO98/26090
[Patent document 8] Japanese Patent Unexamined Publication No. 9-96637
[Patent document 9] Japanese Patent Unexamined Publication No. 11-56395
[Patent document 10] Japanese Patent No. 3686326
[Patent document 11] Japanese Patent Unexamined Publication No. 2005-137360
[Patent document 12] GB1022878

### Disclosure of the Invention

### Object to be Achieved by the Invention

An object of the present invention is to provide a method for conveniently and quickly measuring high-density lipoprotein cholesterol (HDL-C) in a body fluid sample such as blood.

Another object of the present invention is to provide a surfactant suitably usable in such a method mentioned above. More specifically, the object of the present invention is to provide a surfactant having high affinity for aromatic substances and having a marked ability to emulsify and disperse, and solubilize organic compositions such as organic pigments. A further object of the present invention is to provide a surfactant free from a possibility of acting as an environmental hormone.

### Means for Achieving the Object

In order to achieve the aforementioned objects, it is required to provide a surfactant which selectively solubilizes HDL. Therefore, the inventors of the present invention conducted various researches on surfactants, and as a result, they found that if a polycyclic polyglycidol compound was used as a surfactant, instead of a polyoxyethylene type surfactant provided so far, HDL could be selectively solubilized, and also found that cholesterol in HDL could be efficiently measured with high accuracy by using such a surfactant. In particular, it was found that a polyglycidol compound represented by the following general formula (I), obtained by binding a phenyl ether substituted with an alkylphenyl group to the glycidol chain, had potent adsorptive property and affinity for aromatic substances, as well as a remarkable ability to solubilize organic compositions, the compound was useful as a surfactant which did not produce turbidity even if the temperature was elevated, and cholesterol in HDL could be very efficiently measured with high accuracy by using this surfactant. The present invention was accomplished on the basis of the aforementioned findings.

The present invention thus provides a method for measuring high-density lipoprotein cholesterol (HDL-C) in a body fluid sample, wherein a polycyclic polyglycidol compound is used as a high-density lipoprotein (HDL) selective surfactant. As the polycyclic polyglycidol compound, a polyglycidol compound represented by the following general formula (I) is preferred: 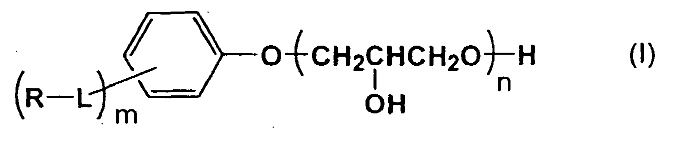 wherein L represents a C₁-C₅ alkylene group, R represents an aryl group (the aryl group may have one or more substituents selected from the group consisting of a C₁-C₁₈ alkyl group, a C₁-C₁₈ alkoxyl group and a halogen atom), m represents an integer of 1 to 5 (when m represents an integer of 2 or larger, the groups represented by R-L-may be the same or different), and n represents an integer of 3 to 20.

Besides the aforementioned polycyclic polyglycidol compound, as preferred embodiments of the aforementioned invention, there are provided the aforementioned method, wherein a surfactant which inhibits dissolution of lipoproteins other than HDL, and/or a coagulant which coagulates lipoproteins other than HDL are used; the aforementioned method, wherein the surfactant which inhibits dissolution of lipoproteins other than HDL is a polyoxyethylene alkyl ether sulfate, an alkylbenzenesulfonate, or a polyoxyethylene/polyoxypropylene condensate; and the aforementioned method, wherein the coagulant which coagulates lipoproteins other than HDL consists of phosphotungstic acid or a salt thereof and a divalent metal ion, dextran sulfate and a divalent metal ion, heparin and a divalent metal ion, or polyoxyethylene.

As other preferred embodiments of the aforementioned invention, there are provided the aforementioned method, wherein a peroxidase and a chromogen are allowed to react on hydrogen peroxide, which is produced from HDL-C by cholesterol esterase and cholesterol oxidase, to perform a color reaction and thereby measure HDL-C; the aforementioned method, wherein 4-aminoantipyrine or a derivative thereof and a Trinder's reagent which couples with 4-aminoantipyrine or a derivative thereof, or a leuco dye is used as the chromogen; and the aforementioned method, wherein HDL-C is measured by using a dry analytic element having at least an adhesive layer and a porous development layer on a water-impermeable support.

From another aspect, the present invention provides the use of a kit for measurement of high-density lipoprotein cholesterol, which comprises a polycyclic polyglycidol compound as a high-density lipoprotein selective surfactant, together with at least one kind of reagent selected from the group consisting of cholesterol esterase, cholesterol oxidase, peroxidase and a chromogen in combination.

From a still further aspect, the present invention provides the use of a polyglycidol compound represented by the aforementioned general formula (I) as a high-density lipoprotein selective surfactant.

According to preferred embodiments of the aforementioned invention, there are provided the aforementioned polyglycidol compound, wherein L is a group represented by -C(R¹)(R²)- (wherein R¹ and R² independently represents hydrogen atom or a C₁-C₄ alkyl group); the aforementioned polyglycidol compound, wherein L is a group represented by -C(R¹)(R²)-, R¹ is hydrogen atom, and R² is hydrogen atom or methyl group; the aforementioned polyglycidol compound, wherein m is an integer of 1 to 3, preferably 2 or 3, most preferably 3; and the aforementioned polyglycidol compound, wherein n is an integer of 5 to 18, preferably an integer of 8 to 16.

The present invention also provides a surfactant containing a polyglycidol compound represented by the aforementioned general formula (I).

The present invention also provides the use of a dry analytic element having at least an adhesive layer and a porous development layer on a water-impermeable support, which contains a polyglycidol compound represented by the aforementioned general formula (I) for measurement of high-density lipoprotein cholesterol.

According to the method of the present invention, it becomes possible to selectively solubilize HDL in lipoproteins by using a polycyclic polyglycidol compound as a surfactant, and as a result, cholesterol in HDL contained in a body fluid sample such as blood can be efficiently, quickly and accurately measured.

The polyglycidol compound represented by the aforementioned general formula (I) provided by the present invention has a characteristic feature that said compound has potent adsorptive property and affinity for aromatic substances, and remarkable ability to solubilize organic compositions. Moreover, the polyglycidol compound of the present invention does not have alkylphenol structure or biphenyl structure, and therefore the compound is unlikely to act as an environmental hormone.

The polyglycidol compound of the present invention is useful as a surfactant which does not produce turbidity even if the temperature is raised, and can be used also as an oil solubilizer or a coating stabilizer, as well as a cleaning agent or a surfactant for biochemistry.

### Brief Description of the Drawings

Fig. 1 shows results of an example of the measurement in a solution system (Example 4).
Fig. 2 shows results of examples of the measurement in solution systems (Example 5, Comparative Example).
Fig. 3 shows results of an example of the measurement using a dry analytic element (Example 6).
Fig. 4 shows results of examples of the measurement using a dry analytic element (Example 7, Comparative Example).

### Best Mode for Carrying out the Invention

The method of the present invention is a method for measuring high-density lipoprotein cholesterol (HDL-C) in a body fluid sample, and characterized in that a polycyclic polyglycidol compound is used as an HDL selective surfactant.

As the body fluid, blood, urine can be used. As the body fluid sample, blood or urine, per se, may be used, or such sample subjected to an appropriate pretreatment may also be used.

In the specification, a "polycyclic polyglycidol compound" means a compound having a polyglycidol chain and a partial structure comprising two or more rings at one end of the polyglycidol chain. Preferred compounds are those comprising a polyglycidol chain and an aryl group bound to one end of the polyglycidol chain, wherein the aryl group has one or more aryl groups bound via a linker if needed. Further preferred compounds are those comprising a polyglycidol chain and an aryl ring bound to one end of the polyglycidol chain, wherein the aryl ring has one or more aryl groups each bound via a linker. In the aforementioned polycyclic polyglycidol compound, benzene ring is preferred as the aryl ring. As the linker, a straight or branched alkylene group can be used. The alkylene group may have a substituent as required, and may contain a hetero atom such as nitrogen atom, oxygen atom or sulfur atom in the main chain. As the aryl group bound to the aryl ring optionally via a linker, phenyl group is preferred, and it is preferred that two or more phenyl groups are bound to the aryl ring via a linker. The phenyl group may have one or more substituents.

In the method of the present invention, the polyglycidol compound represented by the aforementioned general formula (1) can be preferably used as the surfactant.

In the aforementioned general formula (I), L represents a C₁-C₅ alkylene group (the aforementioned carbon number C₁-C₅ represents carbon number of the alkylene chain connecting R and the benzene ring). The alkylene group may be straight or branched. As the methylene unit constituting L, a methylene unit represented by -C(R¹)(R²)- (wherein R¹ and R² independently represent hydrogen atom or a C₁-C₄ alkyl group) is preferred, and an alkylene group containing 1 to 5 of the aforementioned methylene units is preferred. In the aforementioned methylene unit, it is preferred that R¹ is hydrogen atom, and R² is hydrogen atom or methyl group. When the alkylene group contains two or more of the aforementioned methylene units, those methylene units may be the same or different. More specifically, examples include, for example, methylene group, methylmethylene group, ethylene group, methylethylene group, propylene group, butylene group. It is preferred that the alkylene group represented by L contains one methylene unit, and it is more preferred that L is a group represented by -C(R¹)(R²)-. In this case, it is more preferred that R¹ is hydrogen atom, and R² is hydrogen atom or methyl group.

R represents an aryl group. As the aryl group, a monocyclic or condensed polycyclic aromatic hydrocarbon group can be used. For example, phenyl group, naphthyl group, are preferred, and the most preferred is phenyl group. Although the aryl group may have one or more substituents selected from the group consisting of a C₁-C₁₈ alkyl group, a C₁-C₁₈ alkoxyl group, and a halogen atom, the aryl group may be unsubstituted. In the specification, alkyl moiety of the alkyl group or alkoxyl group may be a straight, branched, or cyclic alkyl, or a combination thereof. As the halogen atom, fluorine atom, chlorine atom, bromine atom or iodine atom can be used. Examples of the substituent of the aryl group include, for example, methyl group, ethyl group, n-propyl group, isopropyl group, n-butyl group, tert-butyl group, 2-ethylhexyl group, octyl group, nonyl group, decyl group as alkyl groups, and as the halogen atom, fluorine atom, chlorine atom, bromine atom and the like can be used. When the aryl group has a substituent, position of the substituent is not particularly limited, and when the aryl group has two or more substituents, they may be the same or different.

The group represented by R-L- can exist in a number of 1 to 5 at arbitrary positions on the benzene ring, and when two or more of the groups represented by R-L- exist, they may be the same or different. The number of the group represented by R-L- existing on the benzene ring is preferably 1 to 3, more preferably 2 or 3, particularly preferably 3.

Although n represents an integer of 3 to 20, it is preferably 5 to 18, more preferably 8 to 16.

The polyglycidol compound of the present invention may have one or more asymmetric carbons, and there may be stereoisomers (optical isomers or diastereoisomers) based on the asymmetric carbons. Any of stereoisomers in pure form, arbitrary mixtures of stereoisomers, racemates fall within the scope of the present invention. The polyglycidol compound of the present invention may exist as arbitrary hydrates or solvates, and these substances also fall within the scope of the present invention. Furthermore, the compound of the present invention has m of the groups represented by R-L- at arbitrary positions on the benzene ring (m is an integer of 1 to 5), and arbitrary mixtures of isomers in which substitution positions of m of R-L on the benzene ring are different, and arbitrary mixtures of the compounds in which m are different also fall within the scope of the present invention.

In the polyglycidol compound represented by the aforementioned general formula (1), it is preferred that the group represented by R-L- is unsubstituted benzyl group, and 1 to 3 of such benzyl groups exist at arbitrary positions on the benzene ring. It is also preferred that in one or more of the benzyl groups, methyl group exists on the methylene of the benzyl group. For example, the following polyglycidol compounds are preferred (the substitution positions of the benzyl groups on the benzene ring is not particularly limited in both the compounds).

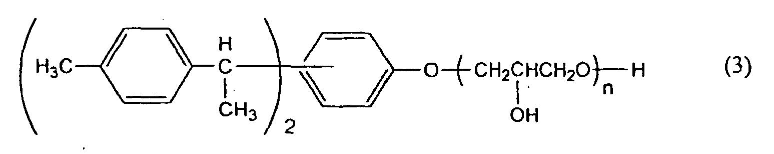

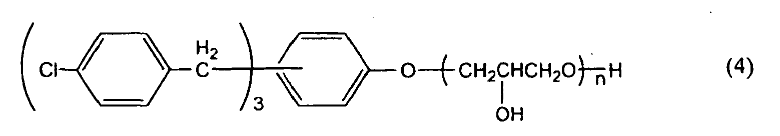

Especially preferred compounds, compounds (5) to compound (10), are shown below (the substitution positions of the benzyl groups on the benzene ring is not particularly limited in all of the compounds).

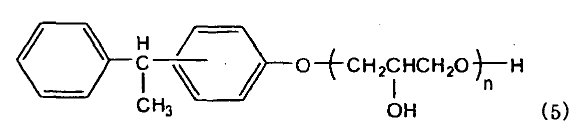

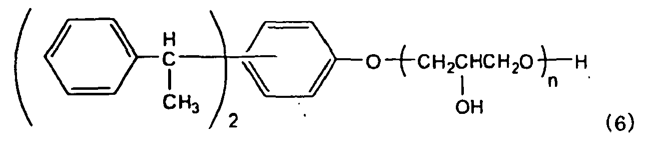

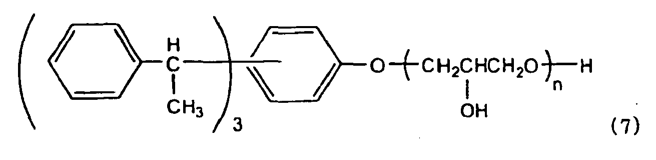

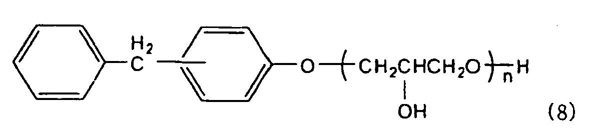

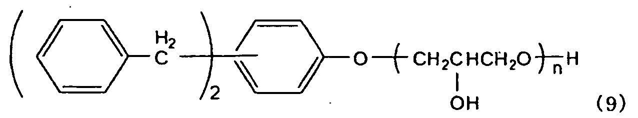

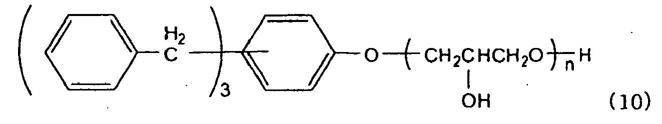

The polyglycidol compound represented by the general formula (I) can be prepared by, for example, adding glycidol to a phenol compound represented by is the following general formula (11): 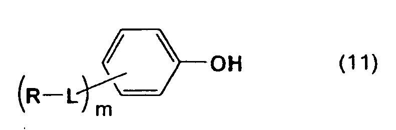 wherein R, L, and m have the same meanings as those defined above, in the presence of a catalyst. In this reaction, by altering a ratio of the phenol compound and glycidol as the starting compounds, it is possible to change glycidol chain length.

Examples of the catalyst include alkaline catalysts, for example, hydroxides of alkali metal or alkaline earth metal such as lithium hydroxide, sodium hydroxide, potassium hydroxide, cesium hydroxide, magnesium hydroxide, calcium hydroxide and barium hydroxide. Sodium hydroxide, potassium hydroxide and cesium hydroxide are preferred, and potassium hydroxide is especially preferred. The amount of the catalyst for use is, for example, 0.0001 to 1%, preferably 0.001 to 0.8%, especially preferably 0.005 to 0.5%, based on the starting compounds. The aforementioned reaction can usually be performed without solvent. The reaction temperature is from room temperature to 200°C, preferably 50 to 200°C, more preferably 100 to 180°C. The reaction time changes depending on the reaction temperature, and is preferably 1 to 150 hours, more preferably 2 to 24 hours, especially preferably 2 to 10 hours.

After the reaction, the reaction mixture can be returned to room temperature, neutralized with an acid, and subjected to conventional treatments to isolate the target substance. Examples of the acid include, for example, mineral acids such as hydrochloric acid, sulfuric acid, nitric acid, hydrobromic acid and phosphoric acid, and organic acids such as methanesulfonic acid, ethanesulfonic acid, acetic acid and p toluenesulfonic acid. Hydrochloric acid, sulfuric acid, phosphoric acid, methanesulfonic acid or acetic acid is preferably used, and it is particularly preferable to use hydrochloric acid, phosphoric acid, methanesulfonic acid or acetic acid. After the neutralization, the target substance can be isolated by filtration when the target substance is water-insoluble. When the target substance is highly viscous, water can be added to obtain the target substance in a state of aqueous solution. The polyglycidol compound of the present invention may be prepared as a mixture of the compounds having different values of "n", and such mixture, per se, can be used as the surfactant according to the method of the present invention, or can also be used for various purposes.

The polyglycidol compound represented by the aforementioned general formula (I) can be used as a surfactant, and can also be used as an emulsifier for emulsion polymerization, emulsifier for metal processing, emulsifier for cosmetics, emulsifier for coating materials, emulsifier for agricultural chemicals, emulsifier for resins, emulsifier for waxes, dispersing agent of pigments, solubilizer for perfumes, household detergent for garments, dishwashing , detergent for industrial use such as detergent for machinery metals, penetrant, wetting agent, antifoam. Furthermore, the compound can also be used as a protein solubilizer, lipid solubilizer, blocking agent, in the field of biochemistry.

In the method of the present invention, a surfactant which suppresses dissolution of lipoproteins other than HDL can be used. Examples of the surfactant which suppresses dissolution of lipoproteins other than HDL include, for example, surfactants selected from polyoxyethylene alkyl ether sulfates, alkylbenzenesulfonates and polyoxyethylene/polyoxypropylene condensates. Examples of the polyoxyethylene alkyl ether sulfates include Aimard 20C (Kao Corp.), and examples of the polyoxyethylene/polyoxypropylene condensates include those of the Pluronic series (Adeka Corp.), .

In the method of the present invention, a coagulant which coagulates lipoproteins other than HDL can be used. Examples of the coagulant which coagulates lipoproteins include systems of a polysaccharide sulfate and an alkaline earth metal ion or a divalent metal ion other than alkaline earth metal, inorganic polyanion salt systems, polyethylene glycol which are well known and described in Nakai T., HDL-Metabolism, Assay Methods and Clinical Application, Chugai Igaku-Sha, 1986 .

Among these precipitation reagents, as the combination systems of a polysaccharide sulfate and a metal ion, precipitation reagents including the dextran sulfate-calcium (2⁺) ion complex described in Journal of Laboratory and Clinical Medicine, 82, 473, 1973, the dextran sulfate-magnesium (2⁺) ion complex described in J. Lipid Res., 11, pp.583-595, 1970; Clin. Chem., 24, pp.931-933, 1978, heparin alone or combination of heparin sodium and manganese ion described in J. Lipid Res., 11, pp.583-595, 1970; Manual of Lipid Operations Lipid Research Clinics Program, Volume I, Pub. No. (NIH), pp.75-628, 1978, combination of heparin, calcium ion and nickel (2⁺) ion described in Japanese Patent Unexamined Publication No. 55-51359, are preferred. As the inorganic polyanion salt system precipitation reagents, combinations of a phosphotungstic acid (salt) and magnesium (2⁺) ion described in J. Lipid Res., 11, pp.583-595, 1970; Clin., Chem., 23, pp.882-884, 1977; Clin., Chem., 25, pp.939-942, 1979; U.S. Patent No. 4,226,713; Japanese Patent Publication No. 63-27659; Japanese Patent Unexamined Publication No. 01-39553, are preferred. More preferred examples include the dextran sulfate-magnesium ion complex.

Examples of the enzyme used in the method of the present invention include, for example, cholesterol esterase and cholesterol oxidase. As the cholesterol esterase, lipase can also be used. Although microorganism as the origin of the enzymes is not particularly limited, as for cholesterol esterase, for example, esterase derived from *Schizophyllum commune* or *Pseudomonas* sp., those derived from other microorganisms can be used. As for cholesterol oxidase, oxidases derived from *Pseudomonas* sp., *Streptmyces* sp., and other microorganisms can be used. The enzymes used in the present invention may be enzymes derived from microorganisms, or recombinant enzymes produced by well-known methods.

Examples of the cholesterol esterase derived from *Schizophyllum commune* include, for example, COE-302 produced by Toyobo Co., Ltd., and examples of cholesterol esterase derived from *Pseudomonas* sp. include COE-311 LPL-312, LPL-314 produced by Toyobo Co., Ltd., CEN produced by Asahi Chemical Co., Ltd.. Examples of the cholesterol oxidase derived from *Pseudomonas* sp. include CHO-PEL, CHO-PEWL produced by KIKKOMAN CORP..

In the method of the present invention, besides these reagents, well-known enzyme reagents, chromogens and pH buffers can be used as reagents for detecting cholesterol.

More specifically, examples of the enzyme include peroxidase, and examples of the chromogen include 4-aminoantipyrine (4-AA), phenol or aniline type Trinder's reagents which induces hydrogen donating coupling to develop color, leuco dyes. As the Trinder's reagents, aniline type reagents can be preferably used, and examples include, for example, N-ethyl-N-sulfopropyl-3-methoxyaniline (ADPS), N-ethyl-N-sulfopropylaniline (ALPS), N-ethyl-N-sulfopropyl-3-methylaniline (TOPS), N-ethyl-N-(2-hydroxy-3-sulfopropyl)-3-methoxyaniline (ADOS), N-ethyl-N-(2-hydroxy-3-sulfopropyl)-3,5-dimethoxyaniline (DAOS), N-(2-hydroxy-3-sulfopropyl)-3,5-dimethoxyaniline (HDAOS), N-ethyl-N-(2-hydroxy-3-sulfopropyl)-3,5-dimethylaniline (MAOS), N-ethyl-N-(2-hydroxy-3-sulfopropyl)-3-methoxyaniline (TOOS) produced by Dojindo Laboratories.

Examples of the pH buffer include carbonates, sulfates, phosphates, pH buffers of Good described in Biochemistry, 5, pp.467-477, 1966,. These pH buffers can be chosen by referring to the descriptions in publications such as Horio T. et al., Basic Experimental Methods for Proteins and Enzymes, Nankodo, 1981; and Biochemistry, 5, pp.467-477, 1966.

Although pH of the buffer can be determined according to optimum pH of enzyme to be used, the pH can be preferably adjusted to be in the range of pH 5.0 to 8.0, more preferably in the range of pH 6.0 to 7.0.

The measurement method of the present invention will be explained as for the method wherein the measurement system is a solution. However, the scope of the present invention is not limited to the following specific embodiment. As for the composition of the reagent solution, a solution having a composition containing the following substances (1) to (6) is preferred.
(1) Cholesterol esterase
(2) Cholesterol oxidase
(3) Surfactant comprising polycyclic polyglycidol compound
(4) Peroxidase
(5) Chromogen (4-AA and Trinder's reagent)
(6) pH Buffer

A reagent solution in a volume of 1 to 1000 µL, preferably 100 to 500 µL, in which concentrations of the above reagents are adjusted to optimum values, is preliminarily incubated at a constant temperature in the range of about 20 to 45°C, preferably in the range of about 30 to 40°C, for 1 to 10 minutes. This reagent solution is added with 0.5 to 50 µL, preferably 1 to 20 µL, of a sample solution, and change of color is measured over time at a wavelength corresponding to color development of the chromogen while the mixture is incubated at a constant temperature. Amount of a test substance in the specimen can be calculated by using a calibration curve prepared beforehand on the basis of the principle of colorimetric assay.

Although required amounts of the enzymes can be suitably determined, all the enzymes, cholesterol esterase, cholesterol oxidase and peroxidase, are each preferably used in an amount, for example, in the range of 0.2 to 20 U/mL, more preferably 1 to 10 U/mL.

When the measurement system is a solution, only the surfactant comprising a polycyclic polyglycidol compound which preferentially dissolves high-density lipoprotein (HDL) may be used as the surfactant, but one or more kinds of other surfactants may also be used in combination if needed. Although concentration of the surfactant is not particularly limited, the surfactant is preferably used at a concentration of, for example, 0.01 to 5%, more preferably 0.1 to 1%.

The measurement method of the present invention using a dry analytic element wherein the measurement system consists of dry reagents will be explained. However, the scope of the present invention is not limited to the following specific embodiment.

The dry analytic element can be constituted so that the element should have at least one adhesive layer and a porous development layer on a water-impermeable support.

The porous layer may be fibrous or non-fibrous, and because said layer functions as a development layer for a liquid sample, it is preferred that said layer has a liquid measuring function. The liquid measuring function means a function of spreading a liquid sample supplied on the surface of the layer by spotting in the layer along the layer surface direction so that the sample should be contained in the layer at a substantially constant rate per unit area without substantial uneven distribution of the components contained in the sample. In order to control development area, development velocity, the hydrophilic polymers or surfactants described in Japanese Patent Unexamined Publication Nos. 60-222770, 63-219397 and 62-182652 can be blended in the development layer, and a polycyclic polyglycidol compound, preferably a polyglycidol compound represented by the aforementioned general formula (I), can also be blended as a surfactant.

The fibrous porous layer is preferably composed of polyester fibers, of which typical examples are those described in Japanese Patent Unexamined Publication Nos. 55-164356, 57-66359, 60-222769. The non-fibrous porous layer is preferably composed of an organic polymer such as polysulfonic acid.

The adhesive layer is a layer having a function of adhering the aforementioned water-impermeable support and the aforementioned porous layer, and hydrophilic polymers such as gelatin and derivatives thereof (for example, phthalated gelatin), cellulose derivatives (for example, hydroxypropylcellulose), agarose, acrylamide polymers, methacrylamide polymers, and copolymers of acrylamide or methacrylamide and various vinyl monomers can be used.

An aqueous solution containing the hydrophilic polymer is uniformly applied by a well-known method, and a known method can be used as the application method. For the application, for example, dip application, extrusion application, doctor blade application, hopper application, curtain application can be suitably chosen and used.

Although the porous layer can also be applied on the adhesive layer, it is preferable to laminate cloth or porous film supplied beforehand as woven fabric. As for the lamination method, adhesion can be attained by a method of uniformly wetting the surface of the adhesive layer containing a hydrophilic polymer with water, superimposing cloth or porous film thereon, and substantially uniformly and lightly applying pressure on them, as described in Japanese Patent Unexamined Publication No. 55-164356. The thickness of the adhesive layer is preferably 0.5 to 50 µm, more preferably 1 to 20 µm.

Preferred materials for light-transmissive support are polyethylene terephthalate, polystyrene and cellulose ethers such as cellulose triacetate. In order to firmly adhere a hydrophilic water absorbing layer, a detection layer and a substantially non-porous reagent layer to the support, the support may usually be provided with an undercoat layer or subjected to a hydrophilization treatment. Although thickness of the support is not particularly limited, it is preferably 10 to 1000 µm, more preferably 300 to 800 µm. In the case of a light-transmissive support, the final detection may be performed on the support side or the porous layer side, but in the case of non-light-transmissive support, the detection is performed on the porous layer side.

Stabilizers, pH buffers, crosslinking agents (hardeners or curing agents), surfactants, polymers may be contained, if needed, and these agents may be contained in the adhesive layer or the porous layer.

The reagent composition for cholesterol measurement and reagent composition exhibiting optical change used for the dry analytic element will be explained.

The reagent composition may be contained in the first porous layer, or may be contained in both the adhesive layer and the porous layer. Alternatively, all or most of the reagent composition may be contained in one of the layers, or the reagent composition may be added to a layer other than the adhesive layer and the porous layer.

In the dry analytic element for HDL-C detection, both the enzymes, cholesterol esterase, and cholesterol oxidase, are each preferably used in an amount of about 0.1 to 30 kU per 1 m². More preferably they can be used in an amount of about 0.5 to 15 kU per 1 m².

Two kinds of the surfactants, i.e., the surfactant which preferentially dissolves HDL such as a polycyclic polyglycidol compound, and the surfactant which inhibits dissolution of lipoproteins other than HDL, can be used each in an amount of about 0.2 to 30 g per 1 m², more preferably in an amount of about 1 to 20 g per 1 m². Ratio of the amounts of the surfactant which preferentially dissolves HDL and the surfactant which inhibits dissolution of lipoproteins other than HDL is preferably about 9/1 to 5/5, and they can be more preferably used at a ratio of 8/2 to 6/4.

As the coagulant for coagulating lipoproteins other than HDL, dextran sulfate and magnesium ion are preferably used, and they are preferably used in an amount of about 0.05 to 10 g per 1 m² as dextran sulfate, and in an amount of about 0.01 to 20 g per 1 m² in terms of magnesium chloride hexahydrate. More preferably, 0.1 to 5 g/m² of dextran sulfate, and 0.5 to 10 g/l m² of magnesium chloride hexahydrate can be used.

Although the origin of peroxidase is not particularly limited, peroxidase derived from horseradish is preferred. As for the amount for use, the peroxidase can be preferably used in an amount of about 1 to 200 kU/m², more preferably about 10 to 100 kU/m².

As the aforementioned chromogen, a combination of 4-aminoantipyrine (4-AA) and the aforementioned reagent which develops color by coupling is preferred, and N-ethyl-N-(2-hydroxy3-sulfopropyl)-3,5-dimethoxyaniline sodium salt (DAOS) can be particularly preferably used. Although the amount of the chromogen to be used is not particularly limited, each of 4-AA and the hydrogen donating coupling agent is preferably used in an amount of, for example, about 0.1 to 10 g/m², more preferably about 0.3 to 5 g/m².

The reagent composition in the dry analytic element for detecting HDL-C may further contain one or more kinds of other additives, such as stabilizers, pH buffers, crosslinking agents (hardeners or curing agents), surfactants and polymers, if needed. These additives may be contained in the adhesive layer and/or the porous layer of the dry analytic element.

pH of the buffer can be determined according to the optimum pH of the enzyme to be used, and can be preferably adjusted to be in the range of pH 5.0 to 8.0. More preferably, the pH can be adjusted to be in the range of pH 6.0 to 7.0.

The dry analytic element can be cut into small pieces in a shape of, for example, square having a side length of about 5 mm to about 30 mm, circle having substantially the same size, folded in a slide frame such as those described in Japanese Patent Publication No. 57-283331, Japanese Utility Model Unexamined Publication Nos. 56-142454, Japanese Patent Unexamined Publication No. 57-63452, Japanese Utility Model Unexamined Publication No. 58-32350, Japanese Patent Unexamined Publication based on International Patent Application (KOHYO) No. 58-501144, and used as a chemical analysis slide. This embodiment is preferred from viewpoints of manufacture, packaging, transportation, storage, measurement operation. Depending on the purpose of use, the element may also have a shape of long tape, and can be stored in a cassette or magazine and used. The element may also be stored as a small piece in a container having an opening and used, or the small piece can be adhered on or stored in a card having an opening and used, or alternatively, the small pieces after the cut, per se, can be used.

When the dry analytic element is used, an aqueous liquid sample solution (for example, body fluid samples such as blood and urine) can be spotted on the porous liquid sample developing layer in a volume in the range of about 2 to 30 µ L, preferably 4 to 15 µL, and the dry analytic element after the spotting can be incubated at a constant temperature in the range of about 20 to 45°C preferably about 30 to 40°C, for 1 to 10 minutes. Color development or color change in the dry analytic element can be measured by reflective photometry from the light-transmissive support side, and amount of a test substance in a specimen can be calculated by using a calibration curve prepared beforehand on the basis of the principle of colorimetric assay.

The measurement operation can be very easily performed by using chemical analysis apparatuses described in, for example, Japanese Patent Unexamined Publication Nos. 60-125543, 60-220862, 61-294367, 58-161867 and highly precise quantitative analysis can be thereby performed. Depending on the purpose or required accuracy, semi-quantitative measurement may be performed by determining degree of coloring by visual inspection.

The dry analytic element can be stored and kept under a dry state before analysis is performed. The element does not require preparation of reagents just before use, and reagents are generally more stable in a dry state. Therefore, the element provides superior convenience and quickness compared with the so-called solution method, which requires preparation of reagent solutions just before use. The element is also excellent in providing a test method with high accuracy and quickness by using a small amount of liquid sample.

### Examples

The present invention will be more specifically explained with reference to examples. However, the scope of the present invention is not limited to the following examples. In the following examples, molecular weight was measured by MALDI-MS, surface tension was measured with a 0.1 mass % aqueous solution, and clouding point was measured in a 1% surfactant and 10% sodium sulfate aqueous solution. HLB was calculated from an organic conceptual diagram. The reactions were performed in a flask made of Pyrex provided with a stirrer and a condenser.

### Example 1

### 1-1) TBG-14

Tribenzylphenol (TBP, (C₆H₅-CH₂)₃-C₆H₃-OH, produced by Sanko Chemical Industry Co., Ltd., containing dibenzylphenol besides the tri-substituted compound as the main component, 86 g) was added with 1.0 g of potassium hydroxide, and the mixture was warmed to 120°C with stirring for mixing under a nitrogen atmosphere. The mixture was slowly added dropwise with 259 g (about 14 equivalents with respect to TBP) of glycidol (produced by Wako Pure Chemical Industries, Ltd.), while the mixture was maintained at a temperature of 120 ± 5°C. The reaction mixture was matured for further 3.5 hours with stirring to obtain the objective substance, TBG-14 (yield: 345 g). This compound had a molecular weight of 586 to 1325 (n = 3 to 13), and hydroxyl value of 592.2 mg KOH/mg. HLB was 13.3, clouding point was higher than 90°C, and the surface tension (0.1 mass % aqueous solution) was 44.8 mN/m.

### 1-2) TBG-10

The synthesis was performed in the same manner as that of the aforementioned method by adding, to TBP (86 g), glycidol (185 g, about 10 equivalents with respect to TBP) and potassium hydroxide (0.8 g) to obtain TBG-10 (271 g). This compound had a molecular weight of 586 to 1473 (n = 3 to 15), hydroxyl value of 556.3 mg KOH/mg, HLB of 11.9, clouding point higher than 90°C, and surface tension of 44.1 mN/m.

### 1-3) TBG-12

The synthesis was performed in the same manner as that of the aforementioned method by adding, to TBP (86 g), glycidol (222 g, about 12 equivalents with respect to TBP) and potassium hydroxide (0.9 g) to obtain TBG-12 (308 g). This compound had a molecular weight of 586 to 1104 (n = 3 to 10), hydroxyl value of 573.7 mg KOH/mg, HLB of 12.7, clouding point higher than 90°C, and surface tension of 44.2 mN/m.

### 1-4) TBG-16

The synthesis was performed in the same manner as that of the aforementioned method by adding, to TBP (72 g), glycidol (249 g, about 16 equivalents with respect to TBP) and potassium hydroxide (1.0 g) to obtain TBG-16 (321 g). This compound had a molecular weight of 586 to 1548 (n = 3 to 16), hydroxyl value of 615.0 mg KOH/mg, HLB of 13.9, clouding point higher than 90°C, and surface tension of 44.7 mN/m.

### Example 2

### 2-1) DSG-12

Distyrylphenol (DSP, [C₆H₅-CH(CH₃)]₂-C₆H₄-OH, produced by Sanko Chemical Industry Co., Ltd., 76 g) was added with 0.9 g of potassium hydroxide, and the mixture was warmed to 120°C with stirring for mixing under a nitrogen atmosphere. The mixture was slowly added dropwise with 222 g (about 12 equivalents with respect to DSP) of glycidol (produced by Wako Pure Chemical Industries, Ltd.), while the mixture was maintained at a temperature of 120 ± 5°C. The reaction mixture was matured with stirring for further 3.5 hours to obtain the objective substance, DSG-12 (298 g). This compound had a molecular weight of 524 to 1042 (n = 3 to 13), hydroxyl value of 602.1 mg KOH/mg, HLB of 13.7, clouding point higher than 90°C, and surface tension of 40.1 mN/m.

### 2-2) DSG-10

The synthesis was performed in the same manner as that of the aforementioned method by adding, to DSP (76 g), glycidol (185 g, about 10 equivalents with respect to DSP) and potassium hydroxide (0.8 g) to obtain DSG-10 (261 g). This compound had a molecular weight of 524 to 1412 (n = 3 to 15), hydroxyl value of 583.1 mg KOH/mg, HLB of 12.9, clouding point higher than 90°C, and surface tension of 39.7 mN/m.

### 2-3) DSG-14

The synthesis was performed in the same manner as that of the aforementioned method by adding, to DSP (73 g), glycidol (249 g, about 14 equivalents with respect to DSP) and potassium hydroxide (1.0 g) to obtain DSG-14 (322 g). This compound had a molecular weight of 524 to 1338 (n = 3 to 14), hydroxyl value of 620.8 mg KOH/mg, HLB of 14.3, clouding point higher than 90°C, and surface tension of 40.3 mN/m.

### Example 3

### 3-1) MSG-8

Monostyrylphenol (MSP, C₆H₅-CB(CH₃)-C₆B₅-OH, produced by Sanko Chemical Industry Co., Ltd., 86 g) was added with 1.0 g of potassium hydroxide, and the mixture was warmed to 120°C with stirring for mixing under a nitrogen atmosphere. The mixture was slowly added dropwise with 237 g (about 8 equivalents with respect to MSP) of glycidol (produced by Wako Pure Chemical Industries, Ltd.), while the mixture was maintained at a temperature of 120 ± 5°C. The reaction mixture was matured with stirring for further 3.5 hours to obtain the objective substance, MSG-8 (323 g). This objective substance had a molecular weight of 420 to 1160 (n = 3 to 13), hydroxyl value of 611.1 mg KOH/mg, HLB of 14.3, clouding point higher than 90°C, and surface tension of 39.7 mN/m.

### 3-2) MSG-10

In the same manner as that of the aforementioned method, MSP (86 g) was added with glycidol (296 g, about 10 equivalents with respect to MSP) and potassium hydroxide (1.1 g) to obtain MSG-10 (382 g). This compound had a molecular weight of 420 to 1234 (n = 3 to 14), hydroxyl value of 630.4 mg KOH/mg, HLB of 15.1, clouding point higher than 90°C, and surface tension of 39.8 mN/m.

### Example 4: Measurement in solution system

A reagent solution having the composition shown below was prepared.

| | |
|---|---|
| MES buffer (pH 7.0) | 360 mmol/L |
| Cholesterol esterase (derived from *Schizophyllum commune*) | 2.4 U/mL |
| Cholesterol oxidase (derived from *Pseudomonas* sp.) | 1.4 U/mL |
| Peroxidase (derived from horseradish) | 38 U/mL |
| 4-Aminoantipyrine (produced Wako Pure Chemical Industries, Ltd.) | 3.0 mmol/L |
| DAOS (produced by the Dojindo Laboratories ) | 4.0 mmol/L |
| Polyglycidol distyrylphenyl ether (n = 10) | 0.62 mg/mL |
| Pluronic F-88 (surfactant, produced by Adeka Corp.) | 0.42 mg/mL |

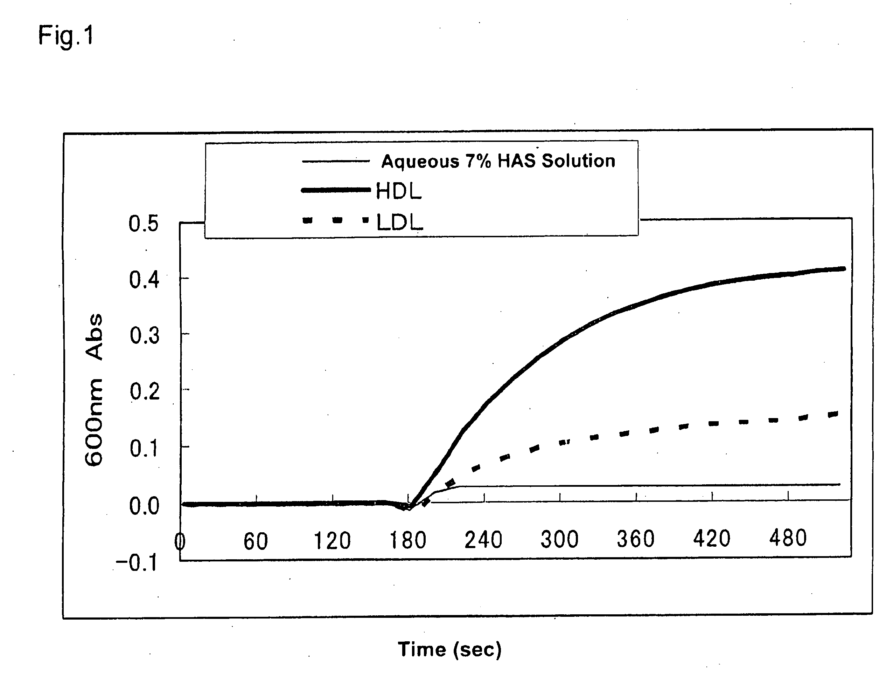

A sample obtained from a purified product of HDL or LDL by adjusting cholesterol concentration thereof to be 100 mg/dL and a 7% human albumin (HSA) aqueous solution were used as specimens. The aforementioned reagent solution (245 µL) was incubated beforehand at 37°C for 3 minutes, and added with 5 µL of each of the aforementioned specimens, and color development was measured at 600 nm. As a result, complete color development was observed with HDL in about 5 minutes as shown in Fig. 1, and thus higher specificity for HDL was confirmed compared with LDL.

### Example 5: Comparative example

Measurement was performed by using the same composition as that of Example 4 and the same specimens as those used in Example 4, provided that the following surfactant was used instead of polyglycidol distyrylphenyl ether (n = 10). Emalgen A90 (polyoxyethylene distyrylphenyl ether): 0.62 mg/mL

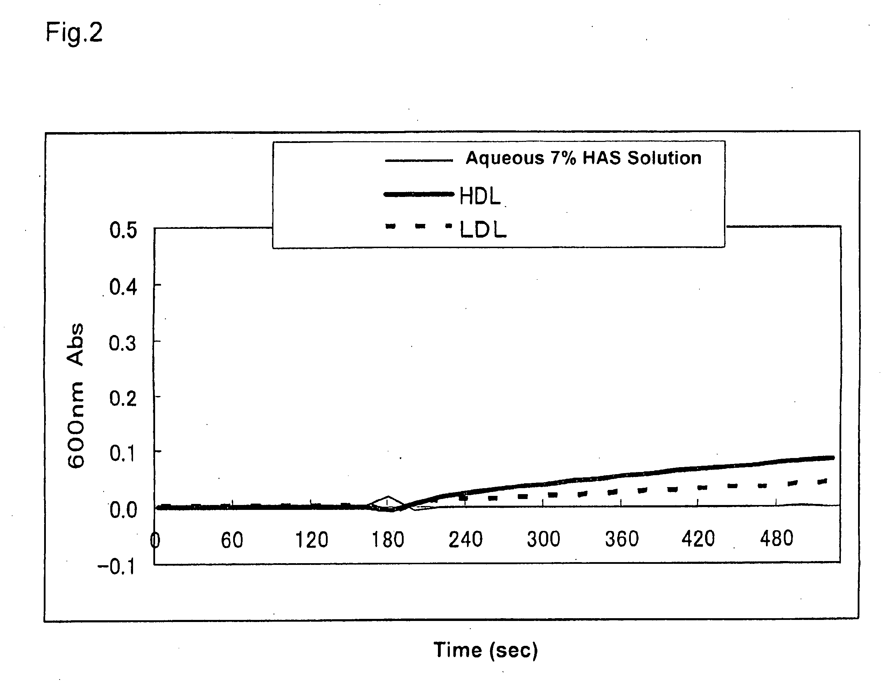

As a result, the surfactant showed low sensitivity for both LDL and HDL as shown in Fig. 2, and also low specificity for HDL.

### Example 6: Dry analytic element

A gelatin aqueous solution was applied in a dry thickness of 14 µm on a smooth colorless transparent 180-µm polyethylene terephthalate film having a gelatin undercoat and dried. The film was wetted by supplying water over the whole surface of the film in an amount of about 30 g/m², then tricot knitting cloth obtained by 36-gauge knitting with 50 denier polyester spun yarns was laminated on the film with lightly applying pressure, and they were dried. Then, an aqueous solution having the following composition was applied on the aforementioned cloth and dried.

| | |
|---|---|
| MES buffer (pH 6.6) | 84 mmol/m² |
| Cholesterol esterase (derived from *Schizophyllum commune*) | 1.8 kU/m² |
| Cholesterol oxidase (derived from *Pseudomonas* sp.) | 4.0 kU/m² |
| Peroxidase | 30 kU/m² |
| 4-Aminoantipyrine (produced Wako Pure Chemical Industries, Ltd.) | 0.4 g/m² |
| DAOS (produced by the Dojindo Laboratories) | 0.4 g/m² |
| Polyglycidol tribenzylphenyl ether (n = 12) | 2.0 g/m² |
| Pluronic F-88 (produced by Adeka Corp.) Dextran sulfate (5,000,000, produced by Wako Pure Chemical | 1.3 g/m² |
| Industries, Ltd.) Magnesium chloride hexahydrate (produced by Wako Pure Chemical Industries, Ltd.) | 0.7 g/m² |
| | 4.6 g/m² |

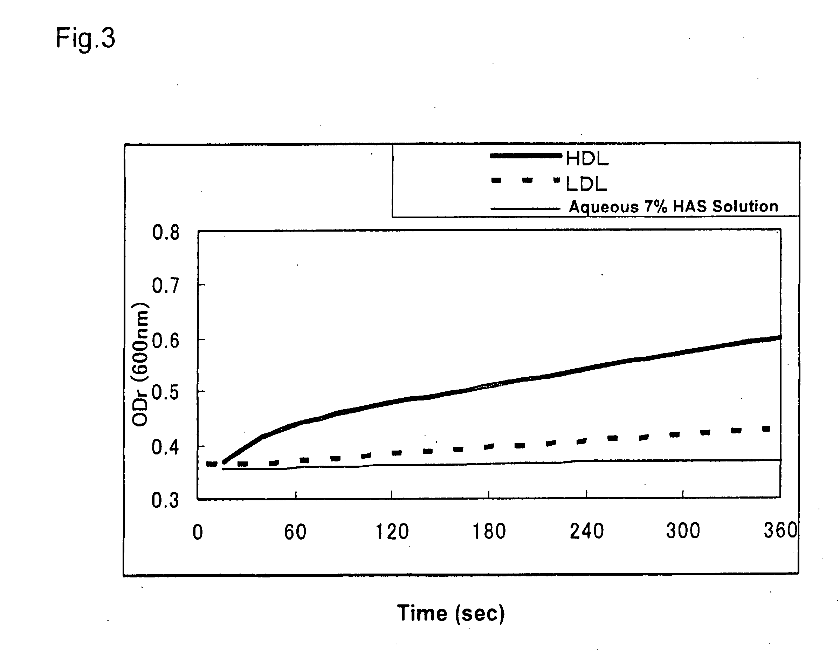

A sample obtained from a purified product of HDL or LDL by adjusting cholesterol concentration thereof to be 100 mg/dL and a 7% human albumin (HSA) aqueous solution were used as specimens. Each specimen (10 µL) was spotted on the aforementioned dry analytic element, and then incubated at 37°C for 6 minutes, and thereafter color development was measured at 600 nm. As a result, complete color development was observed with HDL in about 6 minutes as shown in Fig. 3, whereas ODr was not scarcely elevated with LDL, and thus specificity for HDL could be confirmed.

### Example 7: Comparative Example

A dry analytic element was prepared in the same manner as that of Example 6, provided that the following surfactant was used instead of polyglycidol tribenzylphenyl ether.
Surfactant 10G (polyglycidol nonylphenyl ether, produced by Olin Corp.): 2.0 g/m²

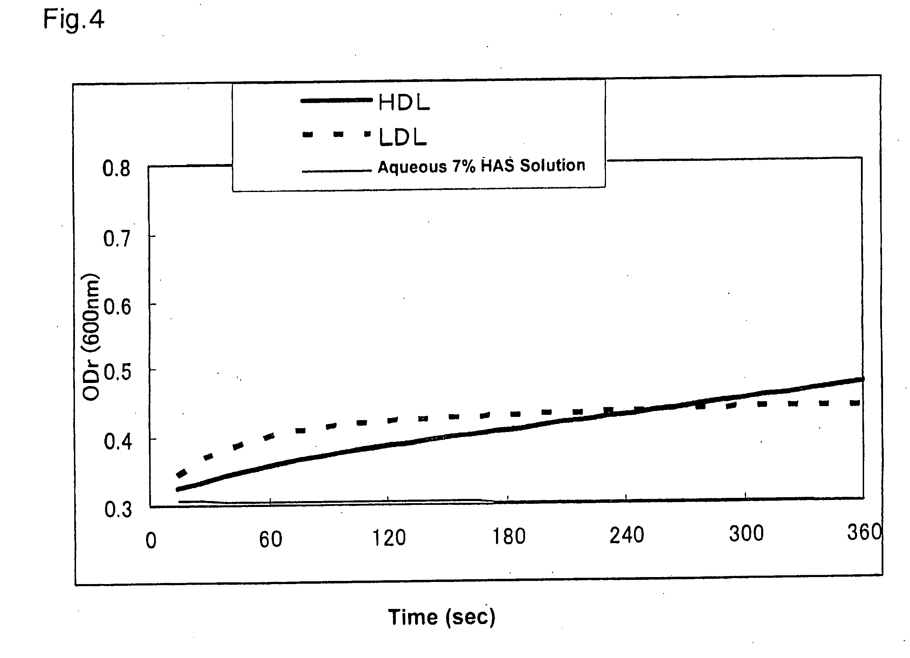

The same specimens as those of Example 6 were spotted, and measurement was performed in the same manner as that of Example 6. It was confirmed that the analytic element prepared by the method described above showed low reactivity for both HDL and LDL, and no HDL specificity, either, as shown in Fig. 4.

## Claims

1. A method for measuring high-density lipoprotein cholesterol in a body fluid sample, wherein a polycyclic polyglycidol compound is used as a high-density lipoprotein selective surfactant.

2. The method according to claim 1, wherein the polycyclic polyglycidol compound is a polyglycidol compound represented by the following general formula (I): 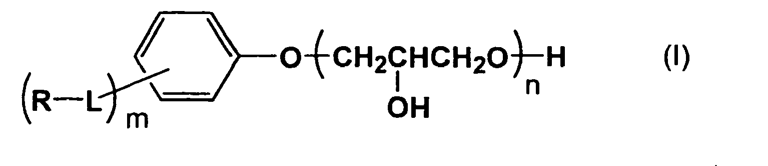 wherein L represents a C₁-C₅ alkylene group, R represents an aryl group (the aryl group may have one or more substituents selected from the group consisting of a C₁-C₁₈ alkyl group, a C₁-C₁₈ alkoxyl group and a halogen atom), m represents an integer of 1 to 5 (when m represents an integer of 2 or larger, the groups represented by R-L-may be the same or different), and n represents an integer of 3 to 20.

3. The method according to claim 1 or 2, wherein a surfactant which inhibits dissolution of lipoproteins other than HDL, and/or a coagulant which coagulates lipoproteins other than HDL are used.

4. The method according to claim 3, wherein the surfactant which inhibits dissolution of lipoproteins other than HDL is a polyoxyethylene alkyl ether sulfate, an alkylbenzenesulfonate, or a polyoxyethylene/polyoxypropylene condensate.

5. The method according to claim 3, wherein the coagulant which coagulates lipoproteins other than HDL consists of phosphotungstic acid or a salt thereof in combination with a divalent metal ion, dextran sulfate in combination with a divalent metal ion, heparin in combination with a divalent metal ion, or polyoxyethylene.

6. The method according to any one of claims 1 to 5, wherein a peroxidase and a chromogen are allowed to react on hydrogen peroxide, which hydrogen peroxide is produced from high-density lipoprotein cholesterol by cholesterol esterase and cholesterol oxidase, in order to perform a color reaction and thereby measure high-density lipoprotein cholesterol.

7. The method according to any one of claims 1 to 6, wherein high-density lipoprotein cholesterol is measured by using a dry analytic element having at least an adhesive layer and a porous development layer on a water-impermeable support.

8. Use of a kit which comprises a polycyclic polyglycidol compound as a high-density lipoprotein selective surfactant, and at least one kind of reagent selected from the group consisting of cholesterol esterase, cholesterol oxidase, peroxidase, and a chromogen in combination for measurement of high-density lipoprotein cholesterol in a body fluid sample.

9. Use of a polyglycidol compound represented by the following general formula (I): 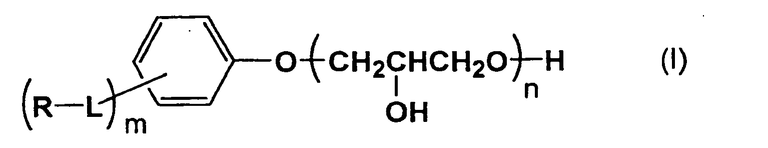 wherein L represents a C₁-C₅ alkylene group, R represents an aryl group (the aryl group may have one or more substituents selected from the group consisting of a C₁-C₁₈ alkyl group, a C₁-C₁₈ alkoxyl group and a halogen atom), m represents an integer of 1 to 5 (when m represents a integer of 2 or larger, the groups represented by R-L- may be the same or different), and n represents an integer of 3 to 20, as a high-density lipoprotein selective surfactant.

10. The use of the polyglycidol compound according to claim 9, wherein L is a group represented by -C(R¹)(R²)- (wherein R¹ and R² independently represents hydrogen atom or a C₁-C₄alkyl group).

11. The use of the polyglycidol compound according to claim 10, wherein R¹ is hydrogen atom, and R² is hydrogen atom or methyl group.

12. The use of the polyglycidol compound according to any one of claims 9 to 11, wherein m is an integer of 1 to 3.

13. The use of the polyglycidol compound according to any one of claims 9 to 12, wherein n is an integer of 5 to 18.

14. Use of a dry analytic element having at least an adhesive layer and a porous development layer on a water-impermeable support, which contains the polyglycidol compound as defined in claims 9 to 13 for measurement of high-density lipoprotein cholesterol.

## Patentansprüche

1. Verfahren zur Messung von High-Density-Lipoprotein-Cholesterol in einer Körperflüssigkeitsprobe, wobei eine polycyclische Polyglycidol-Verbindung als ein für High-Density-Lipoprotein selektives Surfactant verwendet wird.

2. Verfahren nach Anspruch 1, wobei die polycyclische Polyglycidol-Verbindung eine Polyglycidol-Verbindung ist, die durch die folgende allgemeine Formel (I) dargestellt wird: 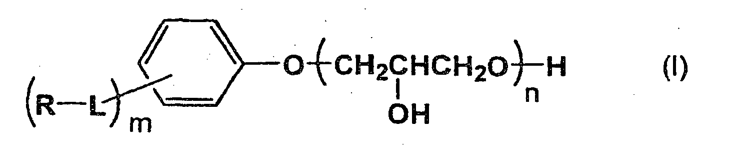 worin L eine C₁-C₅-Alkylengruppe darstellt, R eine Arylgruppe darstellt (die Arylgruppe kann einen oder mehrere Substituenten, ausgewählt aus der Gruppe, bestehend aus einer C₁-C₁₈-Alkylgruppe, einer C₁-C₁₈-Alkoxylgruppe und einem Halogenatom, haben), m eine ganze Zahl von 1 bis 5 darstellt (wenn m eine ganze Zahl von 2 oder größer darstellt, können die durch R-L- dargestellten Gruppen gleich oder unterschiedlich sein) und n eine ganze Zahl von 3 bis 20 darstellt.

3. Verfahren nach Anspruch 1 oder 2, wobei ein Surfactant, das eine Auflösung von anderen Lipoproteinen als HDL inhibiert, und/oder ein Koagulationsmittel, das andere Lipoproteine als HDL koagoniert, verwendet werden/wird.

4. Verfahren nach Anspruch 3, wobei das Surfactant, das eine Auflösung von anderen Lipoproteinen als HDL inhibiert, ein Polyoxyethylenalkylethersulfat, ein Alkylbenzolsulfonat oder ein Polyoxyethylen/Polyoxypropylen-Kondensat ist.

5. Verfahren nach Anspruch 3, wobei das Koagulationsmittel, das andere Lipoproteine als HDL koaguliert, aus Phosphowolframsäure oder einem Salz davon in Kombination mit einem zweiwertigen Metallion, Dextransulfat in Kombination mit einem zweiwertigen Metallion, Heparin in Kombination mit einem zweiwertigen Metallion oder Polyoxyethylen besteht.

6. Verfahren nach einem der Ansprüche 1 bis 5, wobei eine Peroxidase und ein Chromogen mit Wasserstoffperoxid reagieren gelassen werden, wobei das Wasserstoffperoxid aus High-Density-Lipoprotein-Cholesterol durch Cholesterolesterase und Cholesteroloxidase produziert wird, um eine Farbreaktion durchzuführen und dadurch High-Density-Lipoprotein-Cholesterol zu messen.

7. Verfahren nach einem der Ansprüche 1 bis 6, wobei High-Density-Lipoprotein-Cholesterol unter Verwendung eines Trockenanalyseelementes gemessen wird, das wenigstens eine Klebeschicht und eine poröse Entwicklungsschicht auf einem für Wasser undurchlässigen Träger hat.

8. Verwendung eines Kits, der eine polycyclische Polyglycidol-Verbindung als ein für High-Density-Lipoprotein selektives Surfactant und wenigstens eine Art von Reagenz, ausgewählt aus der Gruppe, bestehend aus Cholesterolesterase, Cholesteroloxidase, Peroxidase und einem Chromogen, in Kombination umfasst, zur Messung von High-Density-Lipoprotein-Cholesterol in einer Körperflüssigkeitsprobe.

9. Verwendung einer Polyglycidol-Verbindung, die durch die folgende allgemeine Formel (I) dargestellt wird: 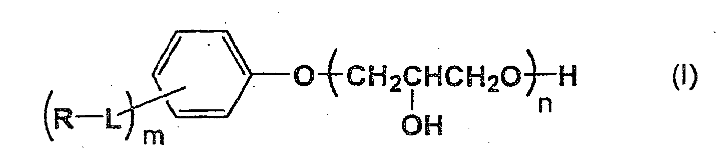 worin L eine C₁-C₅-Alkylengruppe darstellt, R eine Arylgruppe darstellt (die Arylgruppe kann einen oder mehrere Substituenten, ausgewählt aus der Gruppe, bestehend aus einer C₁-C₁₈-Alkylgruppe, einer C₁-C₁₈-Alkoxylgruppe und einem Halogenatom, haben), m eine ganze Zahl von 1 bis 5 darstellt (wenn m eine ganze Zahl von 2 oder größer darstellt, können die durch R-L-dargestellten Gruppen gleich oder unterschiedlich sein) und n eine ganze Zahl von 3 bis 20 darstellt, als ein für High-Density-Lipoprotein selektives Surfactant.

10. Verwendung der Polyglycidol-Verbindung nach Anspruch 9, wobei L eine Gruppe ist, die durch -C(R¹)(R²)- dargestellt wird (worin R¹ und R² unabhängig ein Wasserstoffatom oder eine C₁-C₄-Alkylgruppe darstellen).

11. Verwendung der Polyglycidol-Verbindung nach Anspruch 10, wobei R¹ Wasserstoffatom ist und R² Wasserstoffatom oder Methylgruppe ist.

12. Verwendung der Polyglycidol-Verbindung nach einem der Ansprüche 9 bis 11, wobei m eine ganze Zahl von 1 bis 3 ist.

13. Verwendung der Polyglycidol-Verbindung nach einem der Ansprüche 9 bis 12, wobei n eine ganze Zahl von 5 bis 18 ist.

14. Verwendung eines Trockenanalyseelementes, das wenigstens eine Klebeschicht und eine poröse Entwicklungsschicht auf einem für Wasser undurchlässigen Träger hat, das die Polyglycidol-Verbindung, wie in Ansprüchen 9 bis 13 definiert, enthält, zur Messung von High-Density-Lipoprotein-Cholesterol.

## Revendications

1. Procédé de mesure de cholestérol de lipoprotéine haute densité dans un échantillon de fluide corporel, dans lequel un composé de polyglycidol polycyclique est utilisé comme tensioactif sélectif de lipoprotéine haute densité.

2. Procédé selon la revendication 1, dans lequel le composé de polyglycidol polycyclique est un composé de polyglycidol représenté par la formule générale (I) suivante : 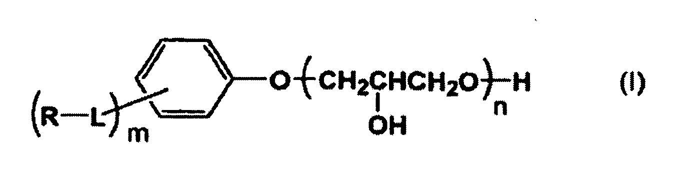 où L représente un groupe alkylène en C₁-C₅, R représente un groupe aryle (le groupe aryle peut présenter un ou plusieurs substituants choisis dans le groupe constitué d'un groupe alkyle en C₁-C₁₈, d'un groupe alcoxyle en C₁-C₁₈ et d'un atome d'halogène), m représente un nombre entier de 1 à 5 (lorsque m représente un nombre entier de 2 ou supérieur, les groupes représentés par R-L- peuvent être identiques ou différents), et n représente un nombre entier de 3 à 20.

3. Procédé selon la revendication 1 ou 2, dans lequel un tensioactif qui inhibe la dissolution de lipoprotéines différentes de HDL, et/ou un coagulant qui coagule des lipoprotéines différentes de HDL sont utilisés.

4. Procédé selon la revendication 3, dans lequel le tensioactif qui inhibe la dissolution de lipoprotéines différentes de HDL est un sulfate d'alkyléther de polyoxyéthylène, un benzènesulfonate d'alkyle ou un condensat de polyoxyéthylène/polyoxypropylène.

5. Procédé selon la revendication 3, dans lequel le coagulant qui coagule des lipoprotéines différentes de HDL est constitué d'acide phosphotungstique ou d'un sel de celui-ci en combinaison avec un ion de métal divalent, de sulfate de dextrane en combinaison avec un ion de métal divalent, d'héparine en combinaison avec un ion de métal divalent, ou de polyoxyéthylène.

6. Procédé selon l'une quelconque des revendications 1 à 5, dans lequel un peroxydase et un chromogène peuvent réagir avec du peroxyde d'hydrogène, lequel peroxyde d'hydrogène est produit à partir de cholestérol de lipoprotéine haute densité par cholestérol estérase et cholestérol oxydase, afin de réaliser une réaction de couleur et de mesurer par-là le cholestérol de lipoprotéine haute densité.

7. Procédé selon l'une quelconque des revendications 1 à 6, dans lequel le cholestérol de lipoprotéine haute densité est mesuré en utilisant un élément analytique sec présentant au moins une couche adhésive et une couche de développement poreux sur un support imperméable à l'eau.

8. Utilisation d'un kit qui comprend un composé de polyglycidol polycyclique comme tensioactif sélectif de lipoprotéine haute densité, et au moins un type de réactif choisi dans le groupe constitué de cholestérol estérase, de cholestérol oxydase, de peroxydase, et d'un chromogène en combinaison pour une mesure du cholestérol de lipoprotéine haute densité dans un échantillon de fluide corporel.

9. Utilisation d'un composé de polyglycidol représenté par la formule générale (I) suivante : 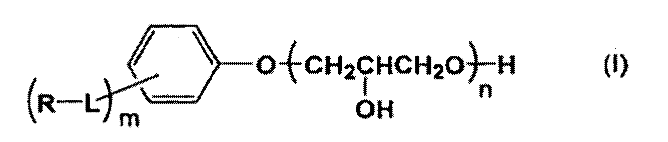 où L représente un groupe alkylène en C₁-C₅, R représente un groupe aryle (le groupe aryle peut présenter un ou plusieurs substituants choisis dans le groupe constitué d'un groupe alkyle en C₁-C₁₈, d'un groupe alcoxyle en C₁-C₁₈ et d'un atome d'halogène), m représente un nombre entier de 1 à 5 (lorsque m représente un nombre entier de 2 ou supérieur, les groupes représentés par R-L- peuvent être identiques ou différents), et n représente un nombre entier de 3 à 20, comme tensioactif sélectif de lipoprotéine haute densité.

10. Utilisation du composé de polyglycidol selon la revendication 9, dans lequel L est un groupe représenté par -C(R¹)(R²)- (où R¹ et R² représentent indépendamment un atome d'hydrogène ou un groupe alkyle en C₁-C₄).

11. Utilisation du composé de polyglycidol selon la revendication 10, dans laquelle R¹ est un atome d'hydrogène, et R² est un atome d'hydrogène ou un groupe méthyle.

12. Utilisation du composé de polyglycidol selon l'une quelconque des revendications 9 à 11, dans laquelle m est un nombre entier de 1 à 3.

13. Utilisation du composé de polyglycidol selon l'une quelconque des revendications 9 à 12, dans laquelle n est un nombre entier de 5 à 18.

14. Utilisation d'un élément analytique sec présentant au moins une couche adhésive et une couche de développement poreux sur un support imperméable à l'eau, lequel contient le composé de polyglycidol comme défini dans les revendications 9 à 13 pour la mesure de cholestérol de lipoprotéine haute densité.
